# EUROPEAN PATENT SPECIFICATION

(11) **EP 2 268 632 B1**
(45) Date of publication and mention of the grant of the patent: **08.08.2012**
(21) Application number: 09731537.8
(22) Date of filing: 27.03.2009
(51) Int. Cl.: C07D 401/12, A61K 31/44, A61P 25/00, A61P 25/16, A61P 25/28, A61P 29/00

(54) **TERTIARY AMINE DERIVATIVES AS PHOSPHODIESTERASE-4 INHIBITORS**
TERTIÄRE AMINDERIVATE ALS PHOSPHODIESTERASE-4-HEMMER
DÉRIVÉS D'AMINES TERTIAIRES COMME INHIBITEURS DE PHOSPHODIESTÉRASE 4

(30) Priority: 14.04.2008 EP 08007284
(43) Date of publication of application: 05.01.2011
(73) Proprietor: CHIESI FARMACEUTICI S.p.A., 43100 Parma (IT)
(72) Inventor: RIZZI, Andrea, I-43100 Parma (IT); ARMANI, Elisabetta, I-43100 Parma (IT); PERETTO, Ilaria, I-43100 Parma (IT); LA PORTA, Elena, I-43100 Parma (IT)
(74) Representative: Minoja, Fabrizio
(86) International application number: PCT/EP2009/002242
(87) International publication number: WO 2009/127320

(56) References cited:
- WO-A-02/074726
- WO-A-2004/009552
- WO-A-2005/061458
- WO-A-2006/135828
- WO-A-2008/006509

## Description

### FIELD OF THE INVENTION

The present invention relates to inhibitors of the phosphodiesterase 4 (PDE4) enzyme. More particularly, the invention relates to tertiary amine derivatives, the processes for preparing such compounds, compositions containing them and the therapeutic use thereof.

### BACKGROUND OF THE INVENTION

The cyclic nucleotide specific phosphodiesterases (PDEs) comprise a family with eleven isoenzymes, known at present, that catalyze the hydrolysis of various cyclic nucleoside monophosphates (including cAMP and cGMP). These cyclic nucleotides act as second messengers within cells and as messengers, carry impulses from cell surface receptors having bound various hormones and neurotransmitters. PDEs regulate the level of cyclic nucleotides within cells and maintain cyclic nucleotide homeostasis by degrading such cyclic mononucleotides resulting in termination of their messenger role.

The isoenzymes can be grouped according to their specificity toward hydrolysis of cAMP or cGMP, their sensitivity to regulation by calcium, calmodulin or cGMP, and their selective inhibition by various compounds.

PDE4 is cAMP specific and its inhibition causes airway relaxation, anti-inflammatory, enhanced cognition and antidepressant activity.

Therefore inhibitors of PDE4 isoenzymes are therapeutic agents which may be useful in treating diseases involving inflammation, such as asthma or arthritis, or diseases of the central nervous such as cognitive decline or memory loss.

Various chemical classes of PDE4 inhibitors are known.

In particular, PDE4 inhibitors belonging to the tertiary amine class have been disclosed in WO 2005/061458 and WO 2006/135828. WO 02/074726 and WO 2004/009552 disclose PDE4 inhibitors having a tertiary amine group directly linked to a pyridine ring. WO 2008/006509 discloses PDE4 inhibitors having a carbonyloxyalkylene group linking a sunstituted pyridyl group to a substituted phenyl group.

However, it is generally known that compounds having IC₅₀ values higher that 1000 nM may show an unsatisfactory therapeutic activity.

As a consequence, the activity of the known PDE4 inhibitors, in particular of those belonging to the tertiary amine class, still requires improvement.

It is therefore object of the present invention to provide compounds belonging to the tertiary amine class, with improved activity over the known PDE4 inhibitors.

### SUMMARY OF THE INVENTION

The invention is directed to compounds acting as inhibitors of the phosphodiesterase 4 (PDE4) enzyme, the processes for preparing such compounds, compositions containing them and the therapeutic use thereof.

In particular the invention is directed to tertiary amines derivatives of general formula (I) 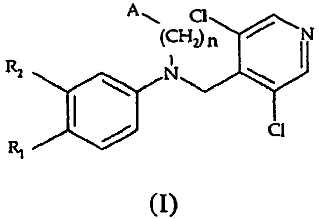 wherein:
n = 1 or 2, preferably 1;
R₁ and R₂ are different or the same and are independently selected from the group consisting of
   - C₁-C₄ alkyloxy;
   - C₃-C₇ cycloalkyloxy; and
   - (C₃-C₇)cycloalkyl-(C₁-C₃)alkyloxy;
and wherein at least one of R₁, and R₂ is C₁-C₄ alkyloxy;
A is an aryl or heteroaryl ring system, having 5 to 10 ring atoms in which at least one ring atom is a heteroatom (e.g. N, S or O), and which is optionally substituted by one or more substituents independently selected from the group consisting of:
   - C₁-C₆ alkyl optionally substituted by one or more C₃-C₇ cycloalkyl;
   - C₂-C₆ alkenyl optionally substituted by one or more C₃-C₇ cycloalkyl;
   - C₂-C₆ alkynyl optionally substituted by one or more C₃-C₇ cycloalkyl;
   - C₃-C₇ cycloalkyl;
   - C₅-C₇ cycloalkenyl;
   - C₃-C₇ cycloalkyloxy;
      wherein in said groups one or more hydrogen atoms can be replaced by halogen atoms;
   - OR₃ wherein R₃ is selected from the group consisting of
      - H;
      - C₁-C₆ alkyl optionally substituted by one or more C₃-C₇ cycloalkyl;
      - C₃-C₇ cycloalkyl,
   wherein in said groups one or more hydrogen atoms can be replaced by halogen atoms;
   - phenyl;
   - benzyl; and
   - NR₄R₅-C₁-C₄ alkyl wherein R₄ and R₅ are each independently H or C₁-C₆ alkyl or together with the nitrogen atom they linked to they form a saturated or partially saturated ring, preferably a piperidyl ring;
   - halogen atoms;
   - CN;
   - NO₂;
   - NR₆R₇ wherein R₆ and R₇ are different or the same and are independently selected from the group consisting of
   - H;
   - C₁-C₆ alkyl, optionally substituted with phenyl;
   - C₁-C₄ alkylsulfonyl;
   - COC₆H₅; and
   - COC₁-C₄ alkyl;
   or together with the nitrogen atom they linked to they form a saturated or partially saturated ring, preferably a piperidyl ring;
   - COR₈ wherein R₈ is OH, NH₂, phenyl or C₁-C₆ alkyl;
   - oxo;
   - HNSO₂R₉ wherein R₉ is C₁-C₄ alkyl or a phenyl optionally substituted with halogen atoms or with a C₁-C₄ alkyl group;
   - SO₂R₁₀ wherein R₁₀ is C₁-C₄ alkyl, OH or NR₆R₇ wherein R₆ and R₇ are as defined above;
   - SOR₁₁ wherein R₁₁ is phenyl or C₁-C₄ alkyl;
   - SR₁₂ wherein R₁₂ is H, phenyl or C₁-C₄ alkyl;
   - COOR₁₃ wherein R₁₃ is H, C₁-C₄ alkyl, phenyl, benzyl and
   - (CH₂)_{q}OR₁₄, wherein q=1, 2, 3 or 4 and R₁₄ is H, C₁-C₄ alkyl or C₁-C₄ cycloalkyl
and pharmaceutically acceptable salts thereof.

The present invention also provides pharmaceutical compositions of compounds of general formula (I) alone or in combination with one or more pharmaceutically acceptable carriers.

In a further aspect the present invention provides the use of compounds of general formula (I) for the preparation of a medicament for the prevention and/or treatment of any disease wherein PDE4 inhibition is required.

The present invention also provides the use of compounds of general formula (I) for preparing a medicament.

In a further aspect, the present invention provides the use of compounds of general formula (I) for the preparation of a medicament for the prevention and/or treatment of an inflammatory disease, disorder or condition characterized by or associated with an undesirable inflammatory immune response or induced by or associated with an excessive secretion of TNF-α and PDE4.

The present invention also provides compounds for use in the treatment of neurological and psychiatric disorders such as Alzheimer's disease, multiple sclerosis, amylolaterosclerosis (ALS), multiple systems atrophy (MSA), schizophrenia, Parkinson's disease, Huntington's disease, Pick's disease, depression, stroke, and spinal cord injury.

### DEFINITIONS

The term "halogen atoms" as used herein includes fluorine, chlorine, bromine, and iodine, preferably chlorine.

As used herein, the expression "linear or branched C₁-Cₓ alkyl" where x is an integer greater than 1, refers to straight and branched chain alkyl groups wherein the number of carbon atoms is in the range 1 to x. Particularly preferred alkyl groups are methyl, ethyl, n-propyl, isopropyl and t-butyl.

Optionally one or more hydrogen atoms in said groups can be replaced by halogen atoms, preferably chlorine or fluorine.

The derived expressions "C₂-C₆ alkenyl" and "C₂-C₆ alkynyl", are to be construed in an analogous manner.

As used herein, the expression "C₃-Cₓ cycloalkyl", where x is an integer greater than 3, refers to cyclic non-aromatic hydrocarbon groups containing from 3 to x ring carbon atoms. Examples include cyclopropyl, cyclobutyl, cyclopentyl, cyclohexyl and cycloheptyl.

Optionally one or more hydrogen atoms in said groups can be replaced by halogen atoms, preferably chlorine or fluorine.

The derived expression "C₅-Cₓ cycloalkenyl", where x is an integer greater than 5, is to be construed in an analogous manner.

As used herein, the expression "ring system" refers to mono- or bicyclic ring systems which may be saturated, partially unsaturated or unsaturated, such as aryl, C₃-C₈ cycloalkyl or heteroaryl, having 5 to 10 ring atoms in which at least one ring atom is a heteroatom (e.g. N, S or O).

Examples of suitable monocyclic systems include thiophene, phenyl and furan. Examples of suitable bicyclic systems include naphthyl and benzothiophene.

### DETAILED DESCRIPTION OF THE INVENTION

The present invention relates to tertiary amine derivatives in which the substituents are an aromatic ring substituted with two alkyloxy groups, an arylmethyl group and a pyridinylmethyl group.

More particularly, the present invention relates to tertiary amines derivatives of general formula (I)

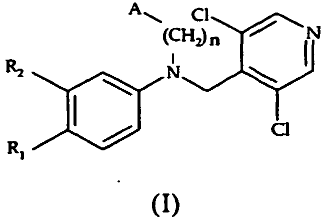

Pharmaceutically acceptable salts include those obtained by reacting the compound with an inorganic or organic acid to form a salt, for example, salts of hydrochloric acid, sulfuric acid, phosphoric acid, methane sulfonic acid, camphor sulfonic acid, oxalic acid, maleic acid, succinic acid and citric acid.

Pharmaceutically acceptable salts also include those in which acidic functions, when present, are reacted with an appropriate base to form, e.g., sodium, potassium, calcium, magnesium, ammonium, and chloride salts.

It has been found that when the phenyl group in A is replaced by the cycloalkyl moiety, the activity falls, in particular in the cell-based assay.

Moreover, from the analysis of the screening results it emerges that hydrogen bond donor or acceptor substituents on the phenyl ring in the A region seem to be preferred, in fact they give rise to compounds showing an improved inhibitory activity in the cell-free assay.

It also been found that compounds in which A is directly linked to the amino nitrogen show an activity higher than 1000 nM in the IC₅₀ PBMCs assay.

In one of the preferred embodiments, R₁ and R₂ are C₁-C₄ alkyloxy.

In a particular embodiment of the invention, A is a heteroaryl ring selected from the group consisting of furan or benzothiophene.

In another particular embodiment of the invention, A is naphthyl.

In one of the preferred embodiments of the invention, A is phenyl.

In one of the preferred embodiment, the optional substituent Rx of the ring system A is selected from the group consisting of C₁-C₆ alkyl, halogen atom, preferably fluorine; SO₂R₁₀ wherein R₁₀ is C₁-C₄ alkyl, preferably methyl or NH₂; CN; OH; COR₈ wherein R₈ is preferably OH; HNSO₂R₉ wherein R₉ is C₁-C₄ alkyl, preferably methyl.

In one of the preferred embodiments of the present invention, Rx is a hydrogen bond donor or acceptor substituent selected from the group consisting of OR₃ wherein R₃ is C₁-C₆ alkyl, preferably methyl or NR₆R₇.

According to a preferred embodiment, the present invention provides the following compounds:

| **Compound** | **Chemical name** |
|---|---|
| **C1** | (3,5-Dichloro-pyridin-4-ylmethyl)-(3,4-dimethoxy-phenyl)-(4-fluorobenzyl)-amine |
| **C2** | 3-{[(3,5-Dichloro-pyridin-4-ylmethyl)-(3,4-dimethoxy-phenyl)-amino]-methyl}-benzonitrile |
| **C3** | (3,5-Dichloro-pyridin-4-ylmethyl)-(3,4-dimethoxy-phenyl)-(3-methoxy-benzyl)-amine |
| **C4** | (3,5-Dichloro-pyridin-4-ylmethyl)-(3,4-dimethoxy-phenyl)-(4-methanesulfonyl-benzyl)-amine |
| **C5** | Benzyl)-(3,5-dichloro-pyridin-4-ylmethyl)-(3,4-dimethoxy-pheny)-amine |
| **C6** | (3,5-Dichloro-pyridin-4-ylmethyl)-(3,4-dimethoxy-phenyl)-phenethyl-amine |
| **C7** | (3,5-Dichloro-pyridin-4-ylmethyl)-(3,4-dimethoxy-benzyl)-(3,4-dimethoxy-phenyl)-amine |
| **C8** | (3,5-Dichloro-pyridin-4-ylmethyl)-(3,4-dimethoxy-phenyl)-(3-fluoro-4-methoxy-benzyl)-amine |
| **C9** | (3,5-Dichloro-pyridin-4-ylmethyl)-(3,4-dimethoxy-phenyl)-furan-2-ylmethyl-amine |
| **C10** | 4-([(3,5-Dichloro-pyridin-4-ylmethyl)-(3,4-dimethoxy-phenyl)-amino]-methyl)-benzenesulfonamide |
| **C11** | 4-([(3,5-Dichloro-pyridin-4-ylmethyl)-(3,4-dimethoxy-phenyl)-amino]-methyl-benzoic acid methyl ester |
| **C12** | N-(3-{[(3,5-Dichloro-pyridin-4-ylmethyl)-(3,4-dimethoxy-phenyl)-amino]-methyl-phenyl)-methanesulfonamide |
| **C13** | (3,5-Dichloro-pyridin-4-ylmethyl)-(3,4-dimethoxy-phenyl)-(4-methoxy-benzyl)-amine |
| **C14** | (3,5-Dichloro-pyridin-4-ylmethyl)-(3-ethoxy-4-methoxy-phenyt)-(4-methoxy-benzyl)-amine |
| **C15** | (3,5-Dichloro-pyridin-4-ylmethyl)-(3-isopropoxy-4-methoxyphenyl)-(4-methoxy-benzyl)-amine |
| **C16** | 4-{[(3,5-dichloro-pyridin-4-ylmethyl)-(3,4-dimethoxy-phenyl)-amino]-methyl}-benzoic acid |
| **C17** | (3,5-Dichloro-pyridin-4-ylmethyl)-(3,4-dimethoxy-phenyl)-thiophen-2-ylmethyl-amine |
| **C18** | Benzo[b]thiophen-2-ylmethyl-(3,5-dichloro-pyridin-4-ylmethyl)-(3,4-dimethoxy-phenyl)-amine |
| **C19** | 3-{[(3,5-Dichloro-pyridin-4-ylmethyl)-(3,4-dimethoxy-phenyl)-amino]-methyl-phenol |

The compounds of general formula (I) may be prepared according to conventional methods. Examples of the processes which can be used are described below and reported in Scheme.

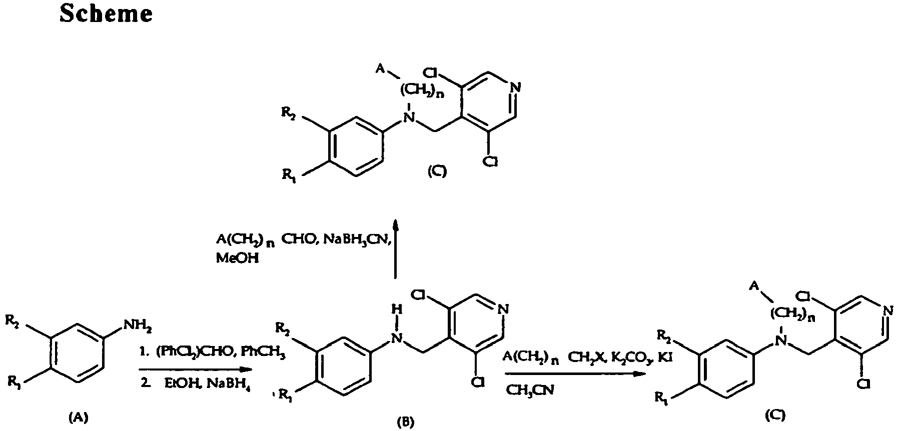

As reported in the above Scheme, the compounds of general formula (I) are prepared according to a process which includes the following steps, the procedure for the preparation of amine of formula (A) being well known:
1^{st} step - functionalization of an amine of formula (A) by reductive amination with an appropriate aldehyde (PhCl₂)-CHO to give a secondary amine of general formula (B).
   The reaction may be carried out for example by formation of the imine intermediate in toluene with molecular sieves, followed by evaporation of the solvent and subsequent reduction of the imine derivative with sodium boron hydride (NaBH₄) in ethanol.
2^{nd} step - further functionalization of the secondary amine of general formula (B), by means of either reductive amination or alkylation with primary alkylating agents to give final compounds of general formula (C).

The present invention also provides pharmaceutical compositions of compounds of general formula (I) in admixture with one or more pharmaceutically acceptable carriers, for example those described in Remington's Pharmaceutical Sciences Handbook, XVII Ed., Mack Pub., N.Y., U.S.A.

Administration of the compounds of the present invention may be accomplished according to patient's needs, for example, orally, nasally, parenterally (subcutaneously, intravenously, intramuscularly, intrasternally and by infusion), by inhalation, rectally, vaginally, topically, locally, transdermally, and by ocular administration. Various solid oral dosage forms can be used for administering compounds of the invention including such solid forms as tablets, gelcaps, capsules, caplets, granules, lozenges and bulk powders. The compounds of the present invention can be administered alone or combined with various pharmaceutically acceptable carriers, diluents (such as sucrose, mannitol, lactose, starches) and known excipients, including but not limited to suspending agents, solubilizers, buffering agents, binders, disintegrants, preservatives, colorants, flavours, lubricants and the like. Time release capsules, tablets and gels are also advantageous.

Various liquid oral dosage forms can also be used for administering compounds of the invention, including aqueous and non-aqueous solutions, emulsions, suspensions, syrups, and elixirs. Such dosage forms can also contain suitable inert diluents known in the art such as water and suitable excipients known in the art such as preservatives, wetting agents, sweeteners, flavours, as well as agents for emulsifying and/or suspending the compounds of the invention. The compounds of the invention may be injected, for example, intravenously, in the form of an isotonic sterile solution. Other preparations are also possible.

Suppositories for rectal administration of the compounds of the present invention can be prepared by mixing the compound with a suitable excipient such as cocoa butter, salicylates and polyethylene glycols.

Formulations for vaginal administration can be in the form of cream, gel, paste, foam, or spray formula containing, in addition to the active ingredient, suitable known carriers.

For topical administration, the pharmaceutical composition can be in the form of creams, ointments, liniments, lotions, emulsions, suspensions, gels, solutions, pastes, powders, sprays, and drops suitable for administration to the skin, eye, ear or nose. Topical administration may also involve transdermal administration via means such as transdermal patches.

The dosages of the compounds of the present invention depend upon a variety of factors including the particular disease to be treated, the severity of the symptoms, the route of administration, the frequency of the dosage interval, the particular compound utilized, the efficacy, toxicology profile, and pharmacokinetic profile of the compound.

Advantageously, the compounds of general formula (I) can be administered for example, at a dosage comprised between 0.001 and 1000 mg/day, preferably between 0.1 and 500 mg/day.

The compounds of general formula (I) may be administered for the prevention and/or treatment of any disease wherein PDE4 inhibition is required. Said disease include: diseases involving inflammation such as asthma and COPD, allergic disease states such as atopic dermatitis, urticaria, allergic rhinitis, allergic conjunctivitis, vernal conjunctivitis, eosinophilic granuloma, psoriasis, inflammatory arthritis, rheumatoid arthritis, septic shock, ulcerative colitis, Crohn's disease, reperfusion injury of the myocardium and brain, chronic glomerulonephritis, endotoxic shock, cystic fibrosis, arterial restenosis, artherosclerosis, keratosis, rheumatoid spondylitis, osteoarthritis, pyresis, diabetes mellitus, pneumoconiosis, toxic and allergic contact eczema, atopic eczema, seborrheic eczema, lichen simplex, sunburn, pruritus in the anogenital area, alopecia areata, hypertrophic scars, discoid lupus erythematosus, systemic lupus erythematosus, follicular and wide-area pyodermias, endogenous and exogenous acne, acne rosacea, Beghet's disease, anaphylactoid purpura nephritis, inflammatory bowel disease, leukemia, multiple sclerosis, gastrointestinal diseases, autoimmune diseases and the like.

They also include neurological and psychiatric disorders such as Alzheimer's disease, multiple sclerosis, amylolaterosclerosis (ALS), multiple systems atrophy (MSA), schizophrenia, Parkinson's disease, Huntington's disease, Pick's disease, depression, stroke, and spinal cord injury.

The present invention will now be further described by way of the following examples.

### EXAMPLE 1

### Preparation of intermediates (A) (Scheme)

### Preparation of (3-ethoxy-4-methoxy-phenyl)-amine (A2)

### Step 1: preparation of 2-ethoxy-1-methoxy-4-nitro-benzene

2-Methoxy-5-nitro-phenol (508 mg, 3 mmoles) is dissolved in DMF (20 mL) under nitrogen atmosphere. K₂CO₃ (900 mg, 6.5 mmoles), KI (490 mg, 2.95 mmol) and ethyl bromide (0.250 mL, 3.3 mmoles) are added and the suspension is heated to 40°C for 28 hours. The mixture is diluted with AcOEt (60 mL) and extracted with 1N NaOH (40 mL) and water (40 mL). The organic layer is dried over Na₂SO₄ and evaporated to dryness. The crude is employed in the next step without purification.

### Step 2: preparation of 3-ethoxy-4-methoxy-aniline

The crude obtained in Step 1 is dissolved in ethanol (99%, 20 mL). Pd/C (10%, 60 mg) and ammonium formate (1.71 g) are added and the resulting mixture is stirred at room temperature for 1 hour. The catalyst is removed by filtration and the solvent is evaporated under reduced pressure. The crude is purified by flash chromatography (SiO₂, petroleum ether/AcOEt from 7/3 to 5/5). The title compound is obtained in amount of 325 mg. The same procedure is applied for the synthesis of (A2) (3-isopropoxy-4-methoxyphenyl)-amine), using suitable reagents.

**Table 1**

| **Compound** | **R₁** | **R₂** | **Analytical characterization** |
|---|---|---|---|
| 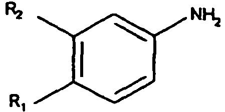 | | | |
| (A1) | OMe | OEt | MS(ESI⁺): 168.1 (MH⁺) |
| (A2) | OMe | OPr | MS(ESI⁺): 182.2 (MH⁺) |

### EXAMPLE 2

### Preparation of intermediates (B) (Scheme)

### Preparation of (3,5-Dichloro-pyridin-4-ylmethyl)-(3,4-dimethoxy-phenyl)-amine (B1)

Commercially available 3,4-dimethoxy-phenyl-amine (1.74 g, 11.3 mmoles) and 3,5-dichloro-pyridine-4-carbaldehyde (2.0 g, 11.3 mmoles) are dissolved in toluene (40 mL) under nitrogen atmosphere. Molecular sieves (4A, 1g) are added and the mixture is heated to reflux. Reaction monitoring is performed by TLC analysis (petroleum ether/ AcOEt 7/3): formation of the imine intermediate is completed after 3 hours. Molecular sieves are removed by filtration and the solvent is evaporated. The residue is dissolved in ethanol (99%, 40 mL). The solution is cooled to 0°C and NaBH₄ (559 mg, 14.7 mmol) is added. The resulting mixture is stirred at room temperature for 18 hours, then water is added (50 mL) and the mixture is extracted AcOEt (3x60 mL). The organic layer is washed with brine, dried over Na₂SO₄ and evaporated to dryness. The crude is purified by flash chromatography (SiO₂, petroleum ether/AcOEt from 9/1 to 7/3). The title compound is obtained as a yellow solid (3.04 g).

The following compounds are prepared following the same synthetic procedure, using suitable reagents:

**Table 2**

| **Compound** | **R₁** | **R₂** | **Analytical characterization** |
|---|---|---|---|
| 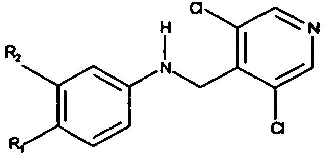 | | | |
| **(B1)** | OMe | OMe | MS(ESI⁺): 313.0 (MH⁺) |
| **(B2)** | OMe | OEt | MS(ES1⁺): 327.1 (MH⁺) |
| **(B3)** | OMe | OPr | MS(ESI⁺): 341.1 (MH⁺) |

### EXAMPLE 3

### Preparation of compounds (C) (Scheme)

### Preparation of (3,5-dichloro-pyridin-4-ylmethyl)-(3,4-dimethoxy-phenyl)-(4-fluoro-benzyl)-amine (C1)

Intermediate B1 (480 mg, 1.5 mmoles) is dissolved in CH₃CN (7.5 mL). Solid K₂CO₃ (518 mg, 3.75 mmoles), solid KI (250 mg, 1.5 mmoles) and neat 4-fluoro-benzyl-bromide (0.191 mL, 1.5 mmoles) are added and the mixture is heated in a sealed vial in a microwave oven at 120°C for 30+30 min. The reaction mixture is diluted with water (40 mL) and extracted with AcOEt (3x40 mL). The organic layer is washed with brine, dried over Na₂SO₄ and evaporated to dryness. The crude is purified by flash chromatography (SiO₂ petroleum ether/AcOEt from 9/1 to 7/3). The title compound is obtained as a light-yellow solid in amount of 407 mg.

The final compounds listed below and reported in Table 3 are prepared according to the same procedure, employing either intermediate B1 or B2, B3, B4 and the appropriate alkylating agents.

The following compounds are prepared following the same synthetic procedure, using suitable reagents:

**Table 3**

| **Compound** | **R₁** | **R₂** | **(CH₂)ₙ** | **A** | **Analytical characterization** |
|---|---|---|---|---|---|
| **C1** | OMe | OMe | n = 1 | 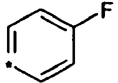 | MS (ESI⁺) 420.9 (MH⁺) (¹H-NMR CDCl₃): 8.40 (s, 2H); 7.15 (dd, 2H); 6.88 (dd, 2H); 6.73 (d, 1H); 6.52-6.47 (m, 2H); 4.58 (s, 2H); 4.30 (s, 2H); 3.80 (s, 3H); 3.74 (s, 3H) |
| **C2** | OMe | OMe | n = 1 | 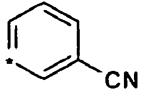 | MS (ESI⁺) 428.0 (MH⁺) (¹H-NMR CDC1₃): 8.41 (s, 2H); 7.52 (s, 1H); 7.44 (m, 2H); 7.30 (dd, 1 H); 6.73 (d, 1H); 6.50 (m, 2H); 4.60 (s, 2H); 4.34 (s, 2H); 3.81 (s, 3 H); 3.76 (s, 3H) |
| **C3** | OMe | OMe | n=1 | 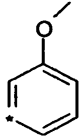 | MS (ESI⁺) 433.1 (MH⁺) (¹H-NMR CDCl₃): 8.40 (s, 2H); 7.12 (dd, 1H); 6.82-6.66 (m, 4H); 6.57-6.49 (m, 2H); 4.64 (s, 2H); 4.35 (s, 2H); 3.80 (s, 3H); 3.74 (s, 3H); 3.73 (s, 3H) |
| **C4** | OMe | OMe | n=1 | 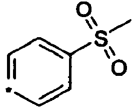 | MS (ESI⁺) 481.1 (MH⁺) (¹H-NMR CDCl₃): 8.41 (s, 2H); 7.77 (d, 2H); 7.42 (d, 2H); 6.72 (d, 1H); 6.57 (d, 1H); 6.50 (dd, 1H); 4.64 (s, 2H); 4.43 (s, 2H); 3.81 (s, 3H); 3.76 (s, 3H); 2.99 (s, 3H) |
| **C5** | OMe | OMe | n = 1 | 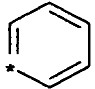 | MS (ESI⁺) 403.1 (MH⁺) (¹H-NMR CDCl₃): 8.40 (s, 2H); 7.27-7.10 (m, 5H); 6.73 (d, 1H); 6.54 (d, 1H); 6.52 (dd, 1H); 4.64 (s, 2H); 4.38 (s, 2H); 3.80 (s, 3H); 3.73 (s, 3H) |
| **C6** | OMe | OMe | n=2 | 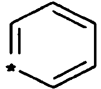 | MS (ESI⁺) 417.1 (MH⁺) (¹H-NMR CDC1₃): 8.44 (s, 2H); 7.29-7.14 (m, 3H); 7.08 (m, 2H); 6.81 (d, 1H); 6.55 (d, 1H); 6.53 (dd, 1H); 4.51 (s, 2H); 3.85 (s, 3H); 3.84 (s, 3H); 3.38 (dd, 2H); 2.76 (dd, 2H) |
| **C7** | OMe | OMe | n = 1 | 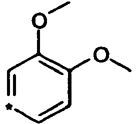 | MS (ESI⁺) 462.1 (MH⁺) (1H-NMR CDCl₃): 7.26 (s, 2H); 6.81-6.67 (m, 4H); 6.60-6.51 (m, 2H); 4.61 (s, 2H); 4.30 (s, 2H); 3.82 (s, 3H); 3.81 (s, 3H); 3.79 (s, 3H); 3.75 (s, 3H) |
| **C8** | OMe | OMe | n = 1 | 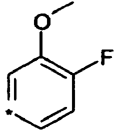 | MS (ESI⁺) 451.1 (MH⁺) (¹H-NMR CDCl₃): 8.41 (s, 2H); 6.93-6.81 (m, 2H); 6.94 (d, 1H); 6.69 (m, 1H); 6.53 (m, 2H); 4.60 (s, 2H); 4.28 (s, 2H); 3.81 (s, 3H); 3.79 (s, 3H); 3.75 (s, 3H) |
| **C9** | OMe | OMe | n = 1 | 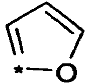 | MS (ESI+) 392.1 (MH+) (1H-NMR CDCl₃): 8.42 (s, 2H); 7.29 (dd, 1H); 6.74 (d, 1H); 6.55 (d, 1H); 6.53 (dd, 1H); 6.23 (dd, 1H); 6.06 (dd, 1H); 4.61 (s, 2H); 4.32 (s, 2H); 3.81 (s, 3H); 3.79 (s, 3H) |
| **C10** | OMe | OMe | n = 1 | 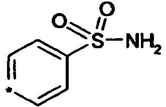 | MS (ESI+) 482.1 (MH+) (1H-NMR CDCl₃): 8.40 (s, 2H); 7.75 (d, 2H); 7.36 (d, 2H); 6.72 (d, 1H); 6.75 (d, 1H); 6.49 (dd, 1H); 4.71 (s br, 2H); 4.63 (s, 2H); 4.40 (s, 2H); 3.81 (s, 3H); 3.76 (s, 3H) |
| **C11** | OMe | OMe | n = 1 | 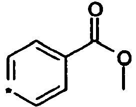 | MS (ESI+) 461.2 (MH+) (1H-NMR CDCl₃): 8.40 (s, 2H); 7.87 (d, 2H); 7.27 (d, 2H); 6.72 (d, 1H); 6.51 (d, 1H); 6.49 (dd, 1H); 4.63 (s, 2H); 4.39 (s, 2H); 3.88 (s, 3H); 3.80 (s, 3H); 3.74 (s, 3H) |
| **C12** | OMe | OMe | n = 1 | 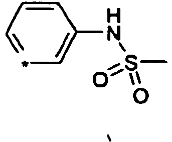 | MS (ESI+) 496.2 (MH+) (1H-NMR CDCl₃): 8.43 (s, 2 H), 6.95 - 7.24 (m, 4 H), 6.72 (d, 1 H), 6.63 (d, 1 H), 6.56 (dd, 1 H), 6.43 (br. s., 1 H), 4.68 (s, 2 H), 4.40 (s, 2 H), 3.80 (s, 3 H), 3.75 (s, 3 H), 2.89 (s, 3 H) |
| **C13** | OMe | OMe | n = 1 | 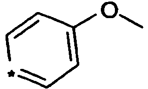 | MS (ESI⁺) 433.1 (MH⁺) (¹H-NMR CDCl₃): 8.39 (s, 2H); 7.10 (d, 2H); 6.73 (m, 3H); 6.50 (m, 2H); 4.58 (s, 2H); 4.29 (s, 2H); 3.80 (s, 3H); 3.75 (s, 3H); 3.74 (s, 3H) |
| **C14** | OMe | OEt | n = 1 | 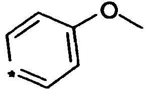 | MS (ESI⁺) 447.1 (MH⁺) (¹H-NMR CDCl₃): 8.41 (s, 2 H), 7.11 (m, 2 H), 6.68 - 6.88 (m, 3 H), 6.43 -6.62 (m, 2 H), 4.59 (s, 2 H), 4.29 (s, 2 H), 3.98 (q, 2 H), 3.81 (s, 3 H), 3.77 (s, 3 H), 1.39 (t, 3 H) |
| **C15** | OMe | OPr | n = 1 | 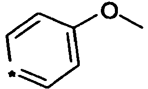 | MS (ESI⁺) 461.0 (MH⁺) (¹H-NMR CDCl₃): 8.40 (s, 2 H), 7.10 (m, 2 H), 6.68 - 6.80 (m, 3 H), 6.47 -6.62 (m, 2 H), 4.58 (s, 2 H), 4.35 (dt, 1 H), 4.27 (s, 2 H), 3.78 (s, 3 H), 3.75 (s, 3 H), 1.26 (d, 6 H) |

The following alkylating agents (as listed in Table 4) employed for the synthesis of the above listed final compounds are not commercially available and are synthesized, according to the following Examples 4, 5, 6.

**Table 4**

| **Alkylating agent** | **structure** |
|---|---|
| **(K1)** | 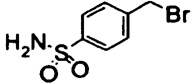 |
| **(K2)** | 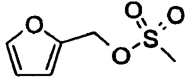 |
| **(K3)** | 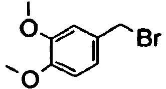 |
| **(K4)** | 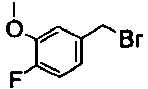 |
| **(K5)** | 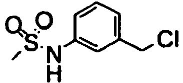 |

### EXAMPLE 4

### Preparation of alkylating agents (K) (Scheme)

### Preparation of 4-bromomethyl-benzene-sulphonamide (K1)

### Step 1: 4-hydroxymethyl-benzene-sulphonamide

LiAlH₄ (235 mg, 6.2 mmoles) is suspended in dry THF (10 mL) under nitrogen atmosphere. The suspension is cooled to 0°C and 4-sulphamoyl-benzoic acid (500 mg, 2.48 mmoles, as a suspension in 10 mL of dry THF) is added. The resulting mixture is then refluxed for 18 hours. The reaction is quenched by addition of 3N HC1 at 0°C. The quenched mixture is extracted with AcOEt, the organic layer is dried over Na₂SO₄ and evaporated to dryness. The crude is purified by flash chromatography (SiO₂, petroleum ether/AcOEt from 9/1 to 1/1) to yield the title compound in amount of 105 mg.

### Step 2: 4-bromomethyl-benzene-sulphonamide

4-Hydroxymethyl-benzene-sulphonamide (0.105 mg, 0.56 mmoles) is dissolved in DCM (5 mL). Polymer supported triphenylphosphine (294 mg, 2.4 mmoles/g, 1.12 mmoles) is added and the mixture is stirred with a shaker at room temperature for 10 minutes. CBr₄ (557 mg, 1.68 mmoles) is then added and stirring is continued for 3 hours. The supported reagent is removed by filtration, the solvent is evaporated and the crude is purified by flash chromatography (SiO₂, petroleum ether/AcOEt 9/1) to yield the title compound as a light-yellow solid (70 mg).

Alkylating agents K3 and K4 are synthesized as described in step 2 of this same Example 4, starting from the corresponding commercially available alcohol derivatives.

### EXAMPLE 5- preparation of intermediates (k) (scheme)

### Preparation of methanesulfonic acid furan-2-ylmethyl ester (K2)

Furan-2-yl-methanol (0.477 mL, 5.5 mmoles) is dissolved in dry DCM (10 mL). The solution is cooled to 0°C and triethylamine (1.16 mL, 8.25 mmoles) and methanesulphonic chloride (0.554 mL, 7.15 mmoles) are added dropwise. The resulting mixture is stirred at room temperature for 3 hours. The suspended solid (triethylamine hydrochloride) is removed by filtration, the filtrate is evaporated to dryness and the crude is employed in the next step without purification.

### Step 2: 4-Bromomethyl-3,5-dichloro-pyridine

(3,5-Dichloro-pyridin-4-yl)-methanol (918 mg, 5.15 mmol) is dissolved in dry DCM (25 mL). Triphenylphosphine (2.70 g, 10.3 mmoles) is added and the mixture is stirred at room temperature for 10 minutes. The solution is then cooled to 0°C and CBr₄ (5.12 g, 15.4 mmoles) is added. The mixture is stirred at room temperature for 30 minutes, then the solvent is evaporated and the crude is purified by flash chromatography (SiO₂, petroleum ether/AcOEt 95/5) to yield 850 mg of the title compound.

### EXAMPLE 6 - preparation of intermediates (k) (scheme)

### Preparation of N-(3-chloromethyl-phenyl)-methane-sulphonamide (K5)

### Step 1: (3-Amino-phenyl)-methanol

3-Amino benzoic acid (1.05 g, 7.65 mmoles) is dissolved in THF (30 mL) under nitrogen atmosphere. Borane (BH₃ 1M solution in THF, 24 ml, 24 mmoles) is added and the resulting solution is stirred at room temperature for 20 hours. The reaction mixture is then poured into a saturated NH₄Cl solution (100 mL) and extracted with AcOEt (3x 100 mL). The organic layer is dried over Na₂SO₄ and evaporated to dryness to yield 388 mg of the title compound, which is employed in the next step without further purification.

### Step 2: N-(3-chloromethyl-phenyl)-methane-sulphonamide

A solution of (3-amino-phenyl)-methanol (388 mg, 3.15 mmoles), lithium chloride (270 mg, 6.3 mmoles) and 2,6-lutidine (0.821 mL, 6.93 mmoles) in DMF (10 mL) is cooled at 0°C under nitrogen atmosphere. Methanesulphonyl chloride (0.536 mL, 6.93 mmoles) is added dropwise and the mixture is stirred at room temperature for 3 hours. Water (30 mL) is then added and the mixture is extracted with AcOEt (3x 40 mL). The organic layer is washed with brine, dried over Na₂SO₄ and evaporated to dryness. The crude is purified by flash chromatography (SiO₂, petroleum ether/AcOEt from 10/0 to 8/2) to yield 330 mg of the title compound.

### EXAMPLE 7

### 4-([(3,5-Dichloro-pyridin-4-ylmethyl)-(3,4-dimethoxy-phenyl)-amino]-methyl)-benzoic acid (C16)

Compound C11 (350 mg, 0.75 mmol) is dissolved in MeOH (14 mL). 1N KOH (3 mL, 3 mmoles) is added and the mixture is refluxed for 1 hour. The solvent is evaporated, the residue is dissolved in water (15 mL) and treated with 3N HCl to pH=1. The solution is then extracted with AcOEt (3x40 mL), the organic layer is dried over Na₂SO₄ and evaporated to dryness. The crude is purified by flash chromatography (SiO₂, AcOEt/petroleum ether from 1/1 to 1/0) to yield the title compound in amount of 230 mg.

**Table 5**

| **Compound** | **R₁** | **R₂** | **(CR₂)ₙ** | **A** | **Analytical characterization** |
|---|---|---|---|---|---|
| **C16** | OMe | OMe | n = 1 | 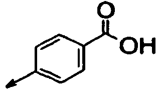 | MS (ES1⁺) 447.1 1 (MH⁺) (¹H-NMR CDCl₃): 8.41(s, 2H); 7.93(d, 2H); 7.31(d, 2H); 6.73(d, 1H); 6.52(m, 2H); 4.64(s, 2H); 4.41(s, 2H); 3.81(s, 3H); 3.75(s, 3H) |

### EXAMPLE 8

### Preparation of the final compounds (C) (Scheme)

### Synthesis of (3,5-dichloro-pyridin-4-ylmethyl)-(3,4-dimethoxy-phenyl)-thiophen-3-ylmethyl-amine

Intermediate B1 (200 mg, 0.64 mmoles) is dissolved in MeOH (10 mL). Molecular sieves (4A, 200 mg) are added and then thiophene-3-carbaldehyde (0.056 mL, 0.64 mmoles). The mixture is stirred at room temperature for 2 hours, then NaBH₃CN (121 mg, 1.9 mmoles) is added. AcOH is added dropwise to pH=5. After 48 hours, molecular sieves are removed by filtration and water is added (20 mL). The mixture is extracted with AcOEt (3x50 mL), the organic layer is washed with brine, dried over Na₂SO₄ and evaporated to dryness. The crude is purified by flash chromatography (SiO₂, petroleum ether/AcOEt from 9/1 to 7/3) to yield the title compound in amount of 173 mg.

Compounds C17, C18 and C19 are prepared following the same synthetic procedure, employing intermediate B1 and appropriate aldehydes, using suitable reagents:

**Table 6**

| **Compound** | **R₁** | **R₂** | **(CH₂)ₙ** | **A** | **Analytical characterization** |
|---|---|---|---|---|---|
| **C17** | OMe | OMe | n = 1 | 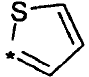 | MS (ESI⁺) 409.1 (MH⁺) (¹H-NMR CDCl₃): 8.42(s, 2H); 7.12(m, 1H); 6.86-6.78(m, 2H); 6.75(d, 1H); 6.59-6.52(m, 2H); 4.58(s, 2H); 4.51(s, 2H); 3.81(s, 3H); 3.78(s, 3H) |
| **C18** | OMe | OMe | n = 1 | 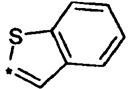 | MS (ESI⁺) 459.4 (MH⁺) (¹H-NMR CDCl₃): 8.44(s, 2H); 7.71 (d, 1H); 7.62(d, 1H); 7.28(dd, 1H); 7.24(dd, 1H); 7.06(s, 1H); 6.75(d, 1H); 6.60(m, 2H); 4.65(s, 2H); 4.60(s, 2H); 3.80(s, 3H); 3.77(s, 3H) |
| **C19** | OMe | OMe | n = 1 | 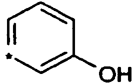 | MS (ESI⁺) 419.1 (MH⁺) (¹H-NMR CDCl₃): 8.42 (s, 2 H), 7.08 (dd, 1 H), 6.67 - 6.82 (m, 3 H), 6.59 - 6.68 (m, 1 H), 6.46 - 6.57 (m, 2 H), 4.64 (s, 2 H), 4.34 (s, 2 H), 3.78 - 3.85 (m, 3 H), 3.72 - 3.79 (m, 3 H) |

| | | | | | |
|---|---|---|---|---|---|
| Legend * NMR s = singlet d = doublet t = triplet q = quartet dd = doublet of doublets m = multiplet br = broad ESI=electrospray | | | | | |

### PHARMACOLOGICAL ACTIVITY

### EXAMPLE 9

### In vitro determination of PDE4 inhibitory activity in the cell free assay

The U937 human monocytic cell line is used as source of PDE4 enzyme. Cells are cultured, harvested and supernatant fraction prepared essentially as described in Torphy TJ et al J. Pharmacol. Exp. Ther. 1992; 263:1195-1205.

PDE4 activity is determined in cells supernatants by assaying cAMP disappearance from the incubation mixtures. 50 µl of cell supernatant are incubated at 30°C for 30 minutes in a final volume of 200 µl in the presence of 1.6 µM cAMP with or without the test compound (50 µl).

The concentration of the test compounds ranges between 10⁻¹² M and 10⁻⁶ M. Reactions are stopped by heat inactivation (2.5 minutes at 100°C) and residual cAMP is measured using an electrochemiluminescence (ECL) -based immunoassay.

The results, expressed as mean ± 95% confidence limits of the molar concentration of the test compound producing 50% inhibition of cAMP disappearance (IC₅₀). The compounds C1-C14 were tested and their values of IC₅₀ in the cell free assay turned out to be comprised between 27 and 807 nM.

Percentage of inhibition of PDE4 activity is calculated, assuming cAMP disappearance in the absence of inhibitors as 100% and cAMP disappearance in heat inactivated samples as 0%.

All the IC₅₀ values of the tested compounds, representative of the invention, were less than 0.2 microM.

### EXAMPLE 10

### In vitro determination of PDE4 inhibitory activity in the peripheral blood mononuclear cells (PBMCs) assay

The assay, which is based on the known inhibitory activity exerted by PDE4 inhibitors on the lipopolysaccharides (LPS)-induced tumour necrosis factor-alpha (TNF-α release in peripheral blood mononuclear cells (PBMCs), is performed according to the method described by Hatzelmann A et al J. Pharmacol. Exp. Ther. 2001; 297:267-279 and by Draheim R et al J. Pharmacol. Exp. Ther. 2004; 308:555-563.

Cryopreserved human PBMCs, (100 µl/well) are incubated in 96-well plates (10⁵ cells/well), for 30 min, in the presence or absence (50 microl) of the test compounds whose concentrations ranged from 10⁻¹² M to 10⁻⁶ M. Subsequently, LPS (3 ng/ml) is added.

After 18 h incubation at 37(C in a humidified incubator under an atmosphere of 95% air and 5% CO2, culture medium is collected and TNF-α measured by ELISA.

The results are expressed as mean ± 95% confidence limits of the molar concentration of the test compound producing 50% inhibition of LPS-induced TNF-α release (IC50).

The effects of the tested compounds are calculated as percent inhibition of TNF-α release, assuming LPS-induced TNF-α production in the absence of inhibitor compound as 100% and basal TNF-α production of PBMCs in the absence of LPS as 0%.

| Compound | IC50 cell free **(nM)** | **IC₅₀ PBMCS (nM)** |
|---|---|---|
| **C1** | 81 | 661 |
| **C3** | 140 | 184 |
| **C4** | 60 | 198 |
| **C9** | 66 | 47.8 |
| **C10** | 38 | 38 |
| **C12** | 54 | - |
| **C13** | 97.1 | 9.85 |
| **C14** | 27 | 117 |

### Comparative examples

Following the teachings of WO 2005/061458 and WO 2006/135828, we have synthesized a comparative molecule corresponding respectively to compound 1 of the invention, from which it differs only in the lack of a methylene bridge between the amino nitrogen and the aryl portion and a comparative molecule similar to compound 5 from which it differs in the lack of a methylene bridge among the amino nitrogen and the aryl portion, which is a cyclopropylmethyl moiety.

Both compounds show values of IC₅₀ PBMCs higher than 1000 nM.

## Claims

1. A compound of general formula (I) 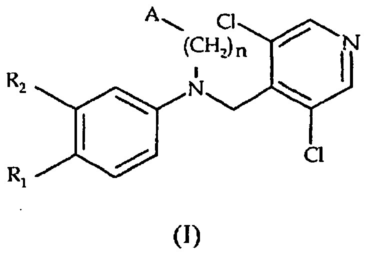 wherein:
n = 1 or 2.
R₁ and R₂ are different or the same and are independently selected from the group consisting of
- C₁-C₄ alkyloxy;
- C₃-C₇ cycloalkyloxy; and
- (C₃-C₇)cycloalkyl-(C₁-C₃)alkyloxy;
and wherein at least one of R₁ and R₂ is C₁-C₄ alkyloxy.
A is an aryl or heteroaryl ring system, having 5 to 10 ring atoms in which at least one ring atom is a heteroatom (e.g. N, S or O), and which is optionally substituted by one or more substituents independently selected from the group consisting of:
- C₁-C₆ alkyl optionally substituted by one or more C₃-C₇ cycloalkyl;
- C₂-C₆ alkenyl optionally substituted by one or more C₃-C₇ cycloalkyl;
- C₂-C₆ alkynyl optionally substituted by one or more C₃-C₇ cycloalkyl;
- C₃-C₇ cycloalkyl;
- C₅-C₇ cycloalkenyl;
- C₃-C₇ cycloalkyloxy, wherein in said groups one or more hydrogen atoms can be replaced by halogen atoms;
- OR₃ wherein R₃ is selected from the group consisting of
- H;
- C₁-C₆ alkyl optionally substituted by one or more C₃-C₇ cycloalkyl;
- C₃-C₇ cycloalkyl;
- phenyl;
- benzyl; and
- NR₄R₅-C₁-C₄ alkyl wherein R₄ and R₅ are each independently H or C₁-C₆ alkyl or they form with the nitrogen atom to which they are linked a saturated or partially saturated ring;
- halogen atoms;
- CN;
- NO₂;
- NR₆R₇ wherein R₆ and R₇ are different or the same and are independently selected from the group consisting of
- H;
- C₁-C₆ alkyl, optionally substituted with phenyl;
- C₁-C₄ alkylsulfonyl;
- COC₆H₅; and
- COC₁-C₄ alkyl;
or they form with the nitrogen atom to which they are linked a saturated or partially saturated ring;
- COR₈ wherein R₈ is OH, NH₂, phenyl or C₁-C₆ alkyl;
- oxo;
- HNSO₂R₉ wherein R₉ is C₁-C₄ alkyl or a phenyl optionally substituted with halogen atoms or with a C₁-C₄ alkyl group;
- SO₂R₁₀ wherein R₁₀ is C₁-C₄ alkyl, OH or NR₆R₇ wherein R₆ and R₇ are as defined above;
- SOR₁₁ wherein R₁₁ is phenyl or C₁-C₄ alkyl;
- SR₁₂ wherein R₁₂ is H, phenyl or C₁-C₄ alkyl;
- COOR₁₃ wherein R₁₃ is H, C₁-C₄ alkyl, phenyl, benzyl and
- (CH₂)_{q}OR₁₄, wherein q=1, 2, 3 or 4 and R₁₄ is H, C₁-C₄ alkyl or C₁-C₄ cycloalkyl and
wherein one or more hydrogen atoms in C₁-C₆ alkyl, C₂-C₆ alkenyl, C₂-C₆ alkynyl, C₃-C₇ cycloalkyl and C₅-C₇ cycloalkenyl can be replaced by halogen atoms
and pharmaceutically acceptable salts thereof.

2. The compound of claim 1 wherein A is an optionally substituted phenyl.

3. The compound of claim 1 wherein A is an optionally substituted heteroaryl ring selected from the group consisting of furan or benzothiophene.

4. The compound of claim 1, selected from:
(3,5-Dichloro-pyridin-4-ylmethyl)-(3,4-dimethoxy-phenyl)-(4-fluoro-benzyl)-amine;
3-{[(3,5-Dichloro-pyridin-4-ylmethyl)-(3,4-dimethoxy-phenyl)-amino]-methyl}-benzonitrile;
(3,5-Dichloro-pyridin-4-ylmethyl)-(3,4-dimethoxy-phenyl)-(3-methoxybenzyl)-amine;
(3,5-Dichloro-pyridin-4-ylmethyl)-(3,4-dimethoxy-phenyl)-(4-methanesulfonyl-benzyl)-amine;
Benzyl-(3,5-dichloro-pyridin-4-ylmethyl)-(3,4-dimethoxy-phenyl)-amine;
(3,5-Dichloro-pyridin-4-ylmethyl)-(3,4-dimethoxy-phenyl)-phenethylamine;
(3,5-Dichloro-pyridin-4-ylmethyl)-(3,4-dimethoxy-benzyl)-(3,4-dimethoxy-phenyl)-amine;
(3,5-Dichloro-pyridin-4-ylmethyl)-(3,4-dimethoxy-phenyl)-(3-fluoro-4-methoxy-benzyl)-amine;
(3,5-Dichloro-pyridin-4-ylmethyl)-(3,4-dimethoxy-phenyl)-furan-2-ylmethyl-amine;
4-{[(3,5-Dichloro-pyridin-4-ylmethyl)-(3,4-dimethoxy-phenyl)-amino]-methyl}-benzenesulfonamide
4-{[(3,5-Dichloro-pyridin-4-ylmethyl)-(3,4-dimethoxy-phenyl)-amino]-methyl}-benzoic acid methyl ester;
N-(3-{[(3,5-Dichloro-pyridin-4-ylmethyl)-(3,4-dimethoxy-phenyl)-amino]-methyl}-phenyl)-methanesulfonamide;
(3,5-Dichloro-pyridin-4-ylmethyl)-(3,4-dimethoxy-phenyl)-(4-methoxybenzyl)-amine;
(3,5-Dichloro-pyridin-4-ylmethyl)-(3-ethoxy-4-methoxy-phenyl)-(4-methoxy-benzyl)-amine;
(3,5-Dichloro-pyridin-4-ylmethyl)-(3-isopropoxy-4-methoxy-phenyl)-(4-methoxy-benzyl)-amine;
4-{[(3,5-Dichloro-pyridin-4-ylmethyl)-(3,4-dimethoxy-phenyl)-amino]-methyl}-benzoic acid;
(3,5-Dichloro-pyridin-4-ylmethyl)-(3,4-dimethoxy-phenyl)-thiophen-2-ylmethyl-amine;
Benzo[b]thiophen-2-ylmethyl-(3,5-dichloro-pyridin-4-ylmethyl)-(3,4-dimethoxy-phenyl)-amine;
3-{[(3,5-Dichloro-pyridin-4-ylmethyl)-(3,4-dimethoxy-phenyl)-amino]-methyl}-phenol.

5. The compound of claim 4, which is (3,5-Dichloro-pyridin-4-ylmethyl)-(3,4-dimethoxy-phenyl)-(4-fluoro-benzyl)-amine (compound 1).

6. The compound of claim 4, which is (3,5-Dichloro-pyridin-4-ylmethyl)-(3,4-dimethoxy-phenyl)-furan-2-ylmethyl-amine (compound 9).

7. The compound of claim 4, which is (3,5-Dichloro-pyridin-4-ylmethyl)-(3,4-dimethoxy-phenyl)-(4-methoxy-benzyl)-amine (compound 13).

8. A pharmaceutical composition comprising the compound of any one of the preceding claims in admixture with one or more pharmaceutically acceptable carriers and/or excipients:

9. A compound of the claims 1 to 7 as a medicament.

10. The use of any of the compounds of the claims 1 to 7 for the preparation of a medicament for the prevention and/or treatment of asthma and COPD, atopic dermatitis, urticaria, allergic rhinitis, allergic conjunctivitis, vernal conjunctivitis, eosinophilic granuloma, psoriasis, inflammatory arthritis, rheumatoid arthritis, septic shock, ulcerative colitis, Crohn's disease, reperfusion injury of the myocardium and brain, chronic glomerulonephritis, endotoxic shock, cystic fibrosis, arterial restenosis, artherosclerosis, keratosis, rheumatoid spondylitis, osteoarthritis, pyresis, diabetes mellitus, pneumoconiosis, toxic and allergic contact eczema, atopic eczema, seborrheic eczema, lichen simplex, sunburn, pruritus in the anogenital area, alopecia areata, hypertrophic scars, discoid lupus erythematosus, systemic lupus erythematosus, follicular and wide-area pyodermias, endogenous and exogenous acne, acne rosacea, Beghet's disease, anaphylactoid purpura nephritis, inflammatory bowel disease, leukemia, gastrointestinal diseases, autoimmune diseases, Alzheimer's disease, multiple sclerosis, amylolaterosclerosis (ALS), multiple systems atrophy (MSA), schizophrenia, Parkinson's disease, Huntington's disease, Pick's disease, depression, stroke, and spinal cord injury.

11. The compounds of claim 1 to 7 for use in the treatment of neurological and psychiatric disorders such as Alzheimer's disease, multiple sclerosis, amylolaterosclerosis (ALS), multiple systems atrophy (MSA), schizophrenia, Parkinson's disease, Huntington's disease, Pick's disease, depression, stroke, and spinal cord injury.

## Patentansprüche

1. Verbindung der allgemeinen Formel (I) 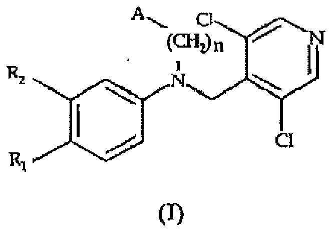 worin:
n= 1 oder 2,
R₁ und R₂ verschieden oder gleich sind und unabhängig ausgewählt sind aus der Gruppe, bestehend aus
- C₁-C₄-Alkyloxy;
- C₃-C₇-Cycloalkyloxy und
- (C₃-C₇) Cycloalkyl- (C₁-C₃) alkyloxy,
und wobei wenigstens eins von R₁ und R₂ C₁-C₄-Alkyloxy ist;
A ein Aryl- oder Heteroarylringsystem ist, das 5 bis 10 Ringatome hat, in dem wenigstens ein Ringatom ein Heteroatom (zum Beispiel N, S oder 0) ist und das gegebenenfalls mit einem oder mehreren Substituenten substituiert ist, die unabhängig ausgewählt sind aus der Gruppe, bestehend aus:
- C₁-C₆-Alkyl, das gegebenenfalls mit einem oder mehreren C₃-C₇-Cycloalkyl substituiert ist;
- C₂-C₆-Alkenyl, das gegebenenfalls mit einem oder mehreren C₃-C₇-Cycloalkyl substituiert ist;
- C₂-C₆-Alkinyl, das gegebenenfalls mit einem oder mehreren C₃-C₇-Cycloalkyl substituiert ist;
- C₃-C₇-Cycloalkyl;
- C₅-C₇-Cycloalkenyl;
- C₃-C₇-Cycloalkyloxy, wobei in den Gruppen ein oder mehrere Wasserstoffatome durch Halogenatome ersetzt sein können;
- OR₃, worin R₃ ausgewählt ist aus der Gruppe, bestehend aus
- H;
- C₁-C₆-Alkyl, das gegebenenfalls mit einem oder mehreren C₃-C₇-Cycloalkyl substituiert ist;
- C₃-C₇-Cycloalkyl;
- Phenyl;
- Benzyl und
- NR₄R₅-C₁-C₄-Alkyl, worin R₄ und R₅ jeweils unabhängig H oder C₁-C₆-Alkyl sind oder mit dem Stickstoffatom, an das sie gebunden sind, einen gesättigten oder teilweise gesättigten Ring bilden;
- Halogenatomen;
- CN;
- NO₂ ;
- NR₆R₇, worin R₆ und R₇ unterschiedlich oder gleich sind und unabhängig ausgewählt sind aus der Gruppe, bestehend aus
- H;
- C₁-C₆-Alkyl, das gegebenenfalls mit Phenyl substituiert ist;
- C₁-C₄-Alkylsulfonyl;
- COC₆H₅ und
- COC₁-C₄-Alkyl;
oder die mit dem Stickstoffatom, an das sie gebunden sind, einen gesättigten oder partiell gesättigten Ring bilden;
- COR₈, worin R₈ OH, NH₂, Phenyl oder C₁-C₆-Alkyl ist;
- Oxo;
- HNSO₂R₉, worin R₉ C₁-C₄-Alkyl oder ein Phenyl ist, das gegebenenfalls mit Halogenatomen oder mit einer C₁-C₄-Alkylgruppe substituiert ist;
- SO₂R₁₀, worin R₁₀ C₁-C₄-Alkyl, OH oder NR₆R₇ ist, worin R₆ und R₇ wie oben definiert sind;
- SOR₁₁, worin R₁₁ Phenyl oder C₁-C₄-Alkyl ist;
- SR₁₂, worin R₁₂ H, Phenyl oder C₁-C₄-Alkyl ist;
- COOR₁₃, worin R₁₃ H, C₁-C₄-Alkyl, Phenyl, Benzyl ist und
- (CH₂)_{q}OR₁₄, worin q=1, 2, 3 oder 4 und R₁₄ H, C₁-C₄-Alkyl oder C₁-C₄-Cycloalkyl ist, und
wobei ein oder mehr Wasserstoffatome in C₁-C₆-Alkyl, C₂-C₆-Alkenyl, C₂-C₆-Alkinyl, C₃-C₇-Cycloalkyl und C₅-C₇-Cycloalkenyl durch Halogenatome ersetzt sein kann/können, und pharmazeutisch verträgliche Salze davon.

2. Verbindung gemäß Anspruch 1, wobei A ein gegebenenfalls substituiertes Phenyl ist.

3. Verbindung gemäß Anspruch 1, wobei A ein gegebenenfalls substituierter Heteroarylring, ausgewählt aus der Gruppe, bestehend aus Furan und Benzothiophen, ist.

4. Verbindung gemäß Anspruch 1, ausgewählt aus:
3,5-Dichlor-pyridin-4-ylmethyl)-(3,4-dimethoxy-phenyl)-(4-fluor-benzyl)-amin;
3-{[(3,5-Dichlor-pyridin-4-ylmethyl)-(3,4-dimethoxy-phenyl)-amino]-methyl}-benzonitril;
(3,5-Dichlor-pyridin-4-ylmethyl)-(3,4-dimethoxy-phenyl)-(3-methoxybenzyl)-amin;
(3,5-Dichlor-pyridin-4-ylmethyl)-(3,4-dimethoxy-phenyl)-(4-methansulfonyl-benzyl)-amin;
Benzyl-(3,5-diehlor-pyridin-4-ylmethyl)-(3,4-dimethoxy-phenyl)-amin;
(3,5-Dichlor-pyridin-4-ylmethyl)-(3,4-dimethoxy-phenyl)-phenethylamin;
(3,5-Dichlor-pyridin-4-ylmethyl)-(3,4-dimethoxy-benzyl)-(3,4-dimethoxy-phenyl)-amin;
(3,5-Dichlor-pyridin-4-ylmethyl)-(3,4-dimethoxy-phenyl)-(3-fluor-4-methoxy-benzyl)-amin;
(3,5-Dichlor-pyridin-4-ylmethyl)-(3,4-dimethoxy-phenyl)-furan-2-ylmethyl-amin;
4-{[(3,5-Dichlor-pyridin-4-ylmethyl)-(3,4-dimethoxy-phenyl)-amino]-methyl}-benzolsulfonamid
4-{[(3,5-Dichlor-pyridin-4-ylmethyl)-(3,4-dimethoxy-phenyl)-amino]-methyl}-benzoesäuremethylester;
N-(3-{[(3,5-Dichlor-pyridin-4-ylmethyl)-(3,4-dimethoxy-phenyl)-amino]-methyl}-phenyl)-methansulfonamid;
(3,5-Dichlor-pyridin-4-ylmethyl)-(3,4-dimethoxy-phenyl)-(4-methoxybenzyl)-amin;
(3,5-Dichlor-pyridin-4-ylmethyl)-(3-ethoxy-4-methoxy-phenyl)-(4-methoxy-benzyl)-amin;
(3,5-Dichlor-pyridin-4-ylmethyl)-(3-isopropoxy-4-methoxyphenyl)-(4-methoxy-benzyl)-amin;
4-{[(3,5-Dichlor-pyridin-4-ylmethyl)-(3,4-dimethoxy-phenyl)-amino]-methyl}-benzoesäure;
(3,5-Dichlor-pyridin-4-ylmethyl)-(3,4-dimethoxy-phenyl)-thiophen-2-ylmethyl-amin;
Benzo[b]thiophen-2-ylmethyl-(3,5-dichlor-pyridin-4-ylmethyl)-(3,4-dimethoxy-phenyl)-amin;
3-{[(3,5-Dichlor-pyridin-4-ylmethyl)-(3,4-dimethoxy-phenyl)-amino]-methyl}-phenol.

5. Verbindung gemäß Anspruch 4, welche (3,5-Dichlor-pyridin-4-ylmethyl)-(3,4-dimethoxy-phenyl)-(4-fluor-benzyl)-amin (Verbindung 1) ist.

6. Verbindung gemäß Anspruch 4, welche (3,5-Dichlor-pyridin-4-ylmethyl)-(3,4-dimethoxy-phenyl)-furan-2-ylmethyl-amin (Verbindung 9) ist.

7. Verbindung gemäß Anspruch 4, welche (3,5-Dichlor-pyridin-4-ylmethyl)-(3,4-dimethoxy-phenyl)-(4-methoxy-benzyl)-amin (Verbindung 13) ist.

8. Pharmazeutische Zusammensetzung, die die Verbindung gemäß einem der vorangehenden Ansprüche im Gemisch mit einem oder mehreren pharmazeutisch verträglichen Trägern und/oder Exzipientien umfasst.

9. Verbindung der Ansprüche 1 bis 7 als Medikament.

10. Verwendung einer der Verbindungen der Ansprüche 1 bis 7 für die Herstellung eines Medikaments zur Prävention und/oder Behandlung von Asthma und COPD, atopischer Dermatitis, Urticaria, allergischer Rhinitis, allergischer Conjunctivitis, Frühjahrs-Conjunctivitis, eosinophilem Granulom, Psoriasis, inflammatorischer Arthritis, rheumatoider Arthritis, septischem Schock, Colitis ulcerosa, Crohn'scher Krankheit, Reperfusionsschädigung des Myokards und des Gehirns, chronischer Glomerulonephritis, endotoxischem Schock, zystischer Fibrose, arterieller Restenose, Arterosklerose, Keratose, rheumatoider Spondilitis, Osteoarthritis, Pyrese, Diabetes mellitus, Pneumoconiose, toxischem und allergischem Kontaktekzem, atopischem Ekzem, seborrhoischem Ekzem, Lichen simplex, Sonnenbrand, Pruritus im anogenitalen Bereich, Alopecia areata, Hypertrophen Narben, Lupus erythematodes discoides, systemischem Lupus erythematodes, follikulären und ausgedehnten Pyodermien, endogener und exogener Akne, Akne rosacea, Beghet-Erkrankung, anaphyloktoider Purpura-Nephritis, inflammatorischer Darmerkrankung, Leukämie, gastrointestinalen Erkrankungen, Autoimmunerkrankungen, Alzheimer'scher Krankheit, Multipler Sklerose, Amylolateralsklerose (ALS), Multisystem-Atrophie (MSA), Schizophrenie, Parkinson'scher Krankheit, Huntington'scher Krankheit, Pick'scher Krankheit, Depression, Schlaganfall und Rückenmarksverletzung.

11. Verbindungen gemäß Anspruch 1 bis 7 zur Verwendung bei der Behandlung von neurologischen und psychiatrischen Störungen, zum Beispiel Alzheimer'scher Krankheit, Multipler Sklerose, Amylolateralsklerose (ALS), Multisystem-Atrophie (MSA), Schizophrenie, Parkinson'scher Krankheit, Huntington'scher Krankheit, Pick'scher Krankheit, Depression, Schlaganfall und Rückenmarksverletzung.

## Revendications

1. Composé de formule générale (I) 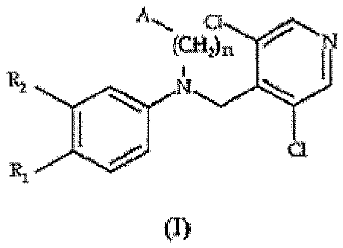 dans laquelle :
n = 1 ou 2.
R₁ et R₂ sont différents ou identiques et sont choisis indépendamment dans le groupe constitué par
- un alkyloxy en C₁-C₄ ;
- un cycloalkyloxy en C₃-C₇ ; et
- un cycloalkyl en C₃-C₇-alkyloxy en C₁-C₃ ;
et dans laquelle au moins un parmi R₁ et R₂ est un alkyloxy en C₁-C₄.
A est un système de cycle aryle ou hétéroaryle, ayant de 5 à 10 atomes de cycle dans lequel au moins un atome de cycle est un hétéroatome (par exemple N, S ou O), et qui est éventuellement substitué par un ou plusieurs substituants choisis indépendamment dans le groupe constitué par :
- un alkyle en C₁-C₆ éventuellement substitué par un ou plusieurs cycloalkyle en C₃-C₇ ;
- un alcényle en C₂-C₆ éventuellement substitué par un ou plusieurs cycloalkyle en C₃-C₇ ;
- un alcynyle en C₂-C₆ éventuellement substitué par un ou plusieurs cycloalkyle en C₃-C₇ ;
- un cycloalkyl en C₃-C₇ ;
- un cycloalcényle en C₅-C₇ ;
- un cycloalkyloxy en C₃-C₇, dans lequel dans lesdits groupes un ou plusieurs atomes d'hydrogène peuvent être remplacés par des atomes d'halogène ;
- un groupe OR₃ dans lequel R₃ est choisi dans le groupe constitué par
- un H ;
- un alkyle en C₁-C₆ éventuellement substitué par un ou plusieurs cycloalkyle en C₃-C₇ ;
- un cycloalkyle en C₃-C₇ ;
- un phényle ;
- un benzyle ; et
- un NR₄R₅-alkyle en C₁-C₄, dans lequel R₄ et R₅ sont chacun indépendamment un H ou un alkyle en C₁-C₆ ou bien ils forment avec l'atome d'azote auquel ils sont liés un cycle saturé ou partiellement saturé ;
- des atomes d'halogène ;
- un CN ;
- un NO₂ ;
- un NR₆R₇ dans lequel R₆ et R₇ sont différents ou identiques et sont choisis indépendamment dans le groupe constitué par
- un H ;
- un alkyle en C₁-C₆, éventuellement substitué par un phényle ;
- un alkylsulfonyle en C₁-C₄ ;
- un COC₆H₅ ; et
- un CO-alkyle en C₁-C₄ ;
ou bien ils forment avec l'atome d'azote auquel ils sont liés un cycle saturé ou partiellement saturé ;
- un COR₈ dans lequel R₈ est un groupe OH, NH₂, phényle ou alkyle en C₁-C₆ ;
- un groupe oxo ;
- un HNSO₂R₉ dans lequel R₉ est un alkyle en C₁-C₄ ou un phényle éventuellement substitué par des atomes d'halogène ou par un groupe alkyle en C₁-C₄ ;
- un SO₂R₁₀ dans lequel R₁₀ est un groupe alkyle en C₁-C₄, OH ou NR₆R₇ dans lequel R₆ et R₇ sont tels que définis ci-dessus ;
- un SOR₁₁ dans lequel R₁₁ est un groupe phényle ou alkyle en C₁-C₄ ;
- un SR₁₂ dans lequel R₁₂ est un H, un groupe phényle ou alkyle en C₁-C₄ ;
- un COOR₁₃ dans lequel R₁₃ est un H, un groupe alkyle en C₁-C₄, phényle, benzyle et - un (CH₂)_{q}OR₁₄, dans lequel q = 1, 2, 3 ou 4 et R₁₄ est un H, un groupe alkyle en C₁-C₄ ou cycloalkyle en C₁-C₄ et
dans lequel un ou plusieurs atomes d'hydrogène dans le groupe alkyle en C₁-C₆, alcényle en C₂-C₆, alcynyle en C₂-C₆, cycloalkyle en C₃-C₇ et cycloalcényle en C₅-C₇ peuvent être remplacés par des atomes d'halogène
et les sels pharmaceutiquement acceptables de celui-ci.

2. Composé selon la revendication 1, dans lequel A est un phényle éventuellement substitué.

3. Composé selon la revendication 1, dans lequel A est un cycle hétéroaryle éventuellement substitué choisi dans le groupe constitué par le furane ou le benzothiophène.

4. Composé selon la revendication 1, choisi parmi :
la (3,5-dichloro-pyridin-4-ylméthyl)-(3,4-diméthoxy-phényl)-(4-fluoro-benzyl)-amine ;
le 3-{[(3,5-dichloro-pyridin-4-ylméthyl)-(3,4-diméthoxy-phényl)-amino]-méthyl}-benzonitrile ;
la (3,5-dichloro-pyridin-4-ylméthyl)-(3,4-diméthoxy-phényl)-(3-méthoxybenzyl)-amine ;
la (3,5-dichloro-pyridin-4-ylméthyl)-(3,4-diméthoxy-phényl)-(4-méthanesulfonyl-benzyl)-amine ;
la benzyl-(3,5-dichloro-pyridin-4-ylméthyl)-(3,4-diméthoxy-phényl)-amine ;
la (3,5-dichloro-pyridin-4-ylméthyl)-(3,4-diméthoxy-phényl)-phénéthylamine ;
la (3,5-dichloro-pyridin-4-ylméthyl)-(3,4-diméthoxy-benzyl)-(3,4-diméthoxy-phényl)-amine ;
la (3,5-dichloro-pyridin-4-ylméthyl)-(3,4-diméthoxy-phényl)-(3-fluoro-4-méthoxy-benzyl)-amine ;
la (3,5-dichloro-pyridin-4-ylméthyl)-(3,4-diméthoxy-phényl)-furan-2-ylméthyl-amine ;
le 4-{[(3,5-dichloro-pyridin-4-ylméthyl)-(3,4-diméthoxy-phényl)-amino]-méthyl}-benzènesulfonamide
l'ester méthylique de l'acide 4-{[(3,5-dichloro-pyridin-4-ylméthyl)-(3,4-diméthoxy-phényl)-amino]-méthyl}-benzoïque ;
le N-(3-{[(3,5-dichloro-pyridin-4-ylméthyl)-(3,4-diméthoxy-phényl)-amino]-méthyl}-phényl)-méthanesulfonamide ;
la (3,5-dichloro-pyridin-4-ylméthyl)-(3,4-diméthoxy-phényl)-(4-méthoxybenzyl)-amine ;
la (3,5-dichloro-pyridin-4-ylméthyl)-(3-éthoxy-4-méthoxy-phényl)-(4-méthoxy-benzyl)-amine ;
la (3,5-dichloro-pyridin-4-ylméthyl)-(3-isopropoxy-4-méthoxy-phényl)-(4-méthoxy-benzyl)-amine ;
l'acide 4-{[(3,5-dichloro-pyridin-4-ylméthyl)-(3,4-diméthoxy-phényl)-amino]-méthyl}-benzoïque ;
la (3,5-dichloro-pyridin-4-ylméthyl)-(3,4-diméthoxy-phényl)-thiophén-2-ylméthyl-amine ;
la benzo[b]thiophén-2-ylméthyl-(3,5-dichloro-pyridin-4-ylméthyl)-(3,4-diméthoxy-phényl)-amine ;
le 3-{[(3,5-dichloro-pyridin-4-ylméthyl)-(3,4-diméthoxy-phényl)-amino]-méthyl}-phénol.

5. Composé selon la revendication 4, qui est la (3,5-dichloro-pyridin-4-ylméthyl)-(3,4-diméthoxy-phényl)-(4-fluoro-benzyl)-amine (composé 1).

6. Composé selon la revendication 4, qui est la (3,5-dichloro-pyridin-4-ylméthyl)-(3,4-diméthoxy-phényl)-furan-2-ylméthyl-amine (composé 9).

7. Composé selon la revendication 4, qui est la (3,5-dichloro-pyridin-4-ylméthyl)-(3,4-diméthoxy-phényl)-(4-méthoxy-benzyl)-amine (composé 13).

8. Composition pharmaceutique comprenant le composé selon l'une quelconque des revendications précédentes en mélange avec un ou plusieurs supports et/ou excipients pharmaceutiquement acceptables.

9. Composé selon les revendications 1 à 7 en tant que médicament.

10. Utilisation de l'un quelconque des composés selon les revendications 1 à 7 pour la préparation d'un médicament destiné à la prévention et/ou au traitement de l'asthme et la BPCO, de la dermatite atopique, l'urticaire, la rhinite allergique, la conjonctivite allergique, la conjonctivite printanière, le granulome éosinophile, le psoriasis, l'arthrite inflammatoire, l'arthrite rhumatoïde, le choc septique, la colite ulcéreuse, la maladie de Crohn, une lésion de reperfusion du myocarde et du cerveau, la glomérulonéphrite chronique, un choc endotoxique, la fibrose kystique, la resténose artérielle, l'athérosclérose, la kératose, la spondylite rhumatoïde, l'arthrose, la pyrexie, le diabète sucré, la pneumoconiose, l'eczéma de contact toxique et allergique, l'eczéma atopique, l'eczéma séborrhéique, le lichen simplex, les coups de soleil, un prurit dans la région anogénitale, la pelade, les cicatrices hypertrophiques, le lupus érythémateux discoïde, le lupus érythémateux systémique, les pyodermites folliculaires et sur une zone étendue, l'acné endogène et exogène , l'acné rosacée, la maladie de Beghet, la néphrite du purpura anaphylactoïde, la maladie inflammatoire intestinale, la leucémie, les maladies gastro-intestinales, les maladies auto-immunes, la maladie d'Alzheimer, la sclérose en plaques, la sclérose latérale amyotrophique (SLA), l'atrophie multisystématisée (MSA), la schizophrénie, la maladie de Parkinson, la maladie d'Huntington, la maladie de Pick, la dépression, l'accident vasculaire cérébral, et une lésion de la moelle épinière.

11. Composés selon les revendications 1 à 7 pour une utilisation dans le traitement des troubles neurologiques et psychiatriques tels que la maladie d'Alzheimer, la sclérose en plaques, la sclérose latérale amyotrophique (SLA), l'atrophie multisystématisée (MSA), la schizophrénie, la maladie de Parkinson, la maladie d'Huntington, la maladie de Pick, la dépression, un accident vasculaire cérébral, et une lésion de la moelle épinière.
